# EUROPEAN PATENT APPLICATION

(11) **EP 0 925 787 A1**
(43) Date of publication of application: **30.06.1999**
(21) Application number: 98905676.7
(22) Date of filing: 26.02.1998
(51) Int. Cl.: A61K 31/557

(54) **DRUGS FOR AMELIORATING PULMONARY CIRCULATION**

(30) Priority: 27.02.1997 JP 4436397
(71) Applicant: TORAY INDUSTRIES, INC., Tokyo 103-8666 (JP)
(72) Inventor: KURUMATANI, Hajimu, Kanagawa 248-0034 (JP); TAMURA, Mitsutaka, Kanagawa 248-0036 (JP)
(74) Representative: Kador & Partner
(86) International application number: JP9800801
(87) International publication number: WO9837895

(57) **Abstract**

A pulmonary circulation improving agent contains a prostaglandin I derivative as an active component, and has the vasodilating action selective to the pulmonary blood vessels. The agent has the effect of improving pulmonary circulation by administration to a patient suffering from the increased pulmonary vascular resistance.

## Description

### Technical Field

The present invention relates to a pulmonary circulation improving agent containing as an active component a prostaglandin I derivative or a salt thereof, and a pulmonary circulation improving method using the agent.

### Background Art

Vasodilators have selectivity to the blood vessels depending upon the types thereof, and for example, a Ca antagonist such as nifedipine or the like, a nitrate agent such as nitroglycerin or the like are known to be highly selective to the coronary artery. However, conventional vasodilators are low selective to the pulmonary blood vessels. It has been reported that, for example, the use for treating pulmonary hypertension causes adverse effects due to a decrease in the systemic blood pressure.

On the other hand, prostaglandin I₂ (PGI₂, prostacyclin) (refer to "Nature" Vol. 268, p. 688, 1976) which is representative of prostaglandin I derivatives is known as a substance having the strong action to inhibit platelet aggregation and the strong action to dilate the peripheral artery. As compounds in which the instability of PGI₂ is significantly improved, Japanese Examined Patent Publication Nos. 2-12226, 2-57548 and 1-53672 disclose PGI₂ derivatives having a skeleton in which the structure of the exoenol ether moiety, which is the characteristic structure of PGI₂, is converted into the inter-m-phenylene type. As other compounds in which the stability of prostaglandin I is improved, ataprost, iloprost, clinprost, ciprostene, naxaprostene, taprostene, cicaprost, pimilprost, CH-169, and CS570 are known [refer to Gendaiiryo-sha, "Review Prostaglandins" No. 1, p. 123 (1994), "New Drugs of Tomorrow" 15-IV-p. 185 (1996), and "New Drugs of Tomorrow" 15-III-p. 551 (1996)]. However, it has been not known yet that these prostaglandin I derivatives have a vasodilating action selective to the pulmonary blood vessels.

As described above, conventional vasodilators are low selective to the pulmonary blood vessels, and it has been reported that the use for treating a patient suffering from the increased pulmonary vascular resistance causes adverse effects such as a headache, emesis, reflex tachycardia, the worsening of right heart failure, and the like.

An object of the present invention is to provide a pulmonary circulation improving agent having less adverse effects and excellent effectiveness and practicability, and a pulmonary circulation improving method.

### Disclosure of Invention

The present invention provides a pulmonary circulation improving agent containing as an active component a prostaglandin I derivative, particularly, a prostaglandin I₂ derivative, and a pulmonary circulation improving method using the agent.

### Brief Description of the Drawings

Fig. 1 shows the results of changes in the pulmonary arterial pressure/systemic blood pressure ratio in Example 1.
Fig. 2 shows the results of changes in the pulmonary vascular resistance/systemic vascular resistance ratio in
Example 1.

### Best Mode for Carrying Out the Invention

As the prostaglandin I derivatives of the present invention, prostaglandin I₁ derivatives, prostaglandin I₂ derivatives, prostaglandin I₃ derivatives, or salts thereof may be used. However, prostaglandin I₂ derivatives and salts thereof are preferably used. More preferably, 4,8-inter-m-phenyleneprostaglandin I₂ derivatives or pharmacologically acceptable salts thereof represented by the following formula (I) are used. 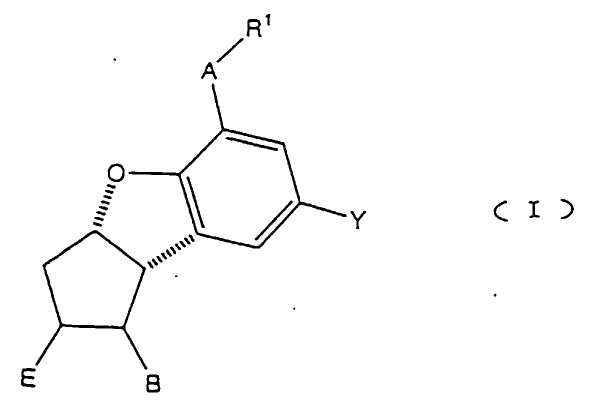 [wherein R¹ is the following:
(A) COOR² wherein R² is:
   1) hydrogen or pharmacologically acceptable cation;
   2) straight chain alkyl having 1 to 12 carbon atoms or branched alkyl having 3 to 14 carbon atoms;
   3) -Z-R³
      wherein Z is a valence bond or straight chain or branched alkylene represented by CₜH₂ₜ wherein t represents an integer of 1 to 6, and R³ is cycloalkyl having 3 to 12 carbon atoms or substituted cycloalkyl having 3 to 12 carbon atoms and substituted by R⁴ which is hydrogen or alkyl having 1 to 5 carbon atoms;
   4) -(CH₂CH₂O)ₙCH₃
      wherein n is an integer of 1 to 5;
   5) -Z-Ar¹
      wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein the substituent is at least one chlorine, bromine, fluorine, iodine, trifluoromethyl, alkyl having 1 to 4 carbon atoms, nitro, cyano, methoxy, phenyl, phenoxy, p-acetoamidobenzamido, -CH=N-NH-C(=O)-NH₂, -NH-C(=O)-Ph, -NH-C(=O)-CH₃ or -NH-C(=O)-NH₂);
   6) -CₜH₂ₜCOOR⁴
      wherein CₜH₂ₜ and R⁴ are defined as the same as the above;
   7) -CₜH₂ₜN(R⁴)₂
      wherein CₜH₂ₜ and R⁴ are defined as the same as the above;
   8) -CH(R⁵)-C(=O)-R⁶
      wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
   9) -CₚH₂ₚ-W-R⁷
      wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
   10) -CH(CH₂OR⁸)₂
      wherein R⁸ is alkyl or acyl having 1 to 30 carbon atoms;
(B) -CH₂OH;
(C) -C(=O)N(R⁹)₂
   wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as the above in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the same as the above in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
   A is the following:
      1) -(CH₂)ₘ-;
      2) -CH=CH-CH₂-;
      3) -CH₂-CH=CH-;
      4) -CH₂-O-CH₂-;
      5) -CH=CH-;
      6) -O-CH₂-; or
      7) -C≡C-;
         wherein m represents an integer of 1 to 3;
   Y is hydrogen, alkyl having 1 to 4 carbon atoms, chlorine, bromine, fluorine, formyl, methoxy or nitro;
   B is -X-C(R¹¹)(R¹²)OR¹³
      wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
      1) -CH₂-CH₂-;
      2) -CH=CH-;
      3) -C≡C-; and
   R¹² is the following:
      1) straight chain alkyl having 1 to 12 carbon atoms, or branched alkyl having 3 to 14 carbon atoms;
      2) -Z-Ar²
         wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
      3) -CₜH₂ₜOR¹⁴
         wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
      4) -Z-R³
         wherein Z and R³ are defined as the same as the above;
      5) -CₜH₂ₜ-CH=C(R¹⁵)R¹⁶
         wherein CₜH₂ₜ is defined as the same as the above, and R¹⁵ and R¹⁶ each represent hydrogen, methyl, ethyl, propyl, or butyl; or
      6) -CᵤH₂ᵤ-C≡C-R¹⁷
         wherein u is an integer of 1 to 7, CᵤH₂ᵤ represents straight chain or branched alkylene, and R¹⁷ represents straight chain alkyl having 1 to 6 carbon atoms;
E is hydrogen or -OR¹⁸
   wherein R¹⁸ represents acyl having 1 to 12 carbon atoms, aroyl having 7 to 15 carbon atoms, or R² (wherein R² is defined as the same as the above); and
   the formula represents the d, l or dl form].

Preferable examples of prostaglandin I derivatives of the present invention include beraprost or salts thereof represented by the following formula (I): 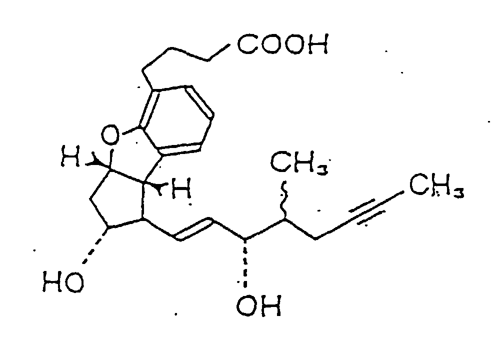 ataprost, iloprost, clinprost, ciprostene, naxaprostene, taprostene, cicaprost, pimilprost, CH-169, CS570, and the like. However, the derivatives are not limited to these compounds.

The prostaglandin I derivatives of the present invention can be synthesized by a known method. For example, compounds represented by formula (I) or salts thereof can be synthesized by the method disclosed in Japanese Examined Patent Publication No. 1-53672.

The pulmonary circulation improving agent of the present invention has the dilating action selective to the pulmonary blood vessels, and is effective as an agent for curing diseases which cause an increase in the pulmonary vascular resistance. The agent also exhibits effectiveness for an increase in the pulmonary vascular resistance which occurs after surgery. Furthermore, the agent selectively decreases the pulmonary vascular resistance as the right heart afterload, and is thus effective as an agent for curing right heart failure.

Diseases which cause an increase in the pulmonary vascular resistance include congenital heart diseases such as Eisenmenger syndrome; diseases causing hypoxemia, such as adult respiratory distress syndrome (ARDS); diseases causing an organic change, such as pulmonary fibrosis; thrombotic diseases of the pulmonary blood vessels, such as pulmonary embolism; pulmonary hypertension such as primary pulmonary hypertension, pulmonary hypertension as a complication of collagen disease; and the like.

The present invention also provides the pulmonary circulation improving method comprising administering a patient suffering from an increase in the pulmonary vascular resistance with an agent containing as an active ingredient the above prostaglandin I derivative(s). As an administration method, a prostaglandin I derivative is administered 1 to 3 times a day in a dose of 0.01 to 100 mg/adult.

Although the pulmonary circulation improving agent of the present invention may contain at least one prostaglandin I derivative, the agent can also be orally administered in the form of a solid containing the additives below.

Examples of such additives include an excipient such as starch, lactose, sucrose, glucose, mannitol, calcium carbonate, calcium sulfate, or the like; a binder such as starch, dextrin, gum arabic, tragacanth, methyl cellulose, gelatin, polyvinyl pyrrolidone, polyvinyl alcohol, or the like; a disintegrator such as starch, polyvinyl pyrrolidone, crystalline cellulose, or the like; a lubricant such as magnesium stearate, talc, or the like; a colorant; a flavor; and the like.

The prostaglandin I derivatives of the present invention can be used in various forms. Examples of the forms include generally used forms such as a tablet, a sugar-coated tablet, a powder, granules, a troche capsule, a pill, a syrup, and the like.

The prostaglandin I derivatives may be parenterally administered in the form of a sterilized solution, and another solute such as sodium chloride, glucose, or the like can also be used in an amount sufficient for making the solution isotonic.

The pulmonary circulation improving agent of the present invention can be applied to the above oral formulations and a variety of other parenteral formulations such as various injections, suppositories, and the like.

### [Examples]

Although the present invention is described in detail below with reference to an example, the present invention is not limited to this example.

### Example 1

Test of comparison with other vasodilating agents in dog:

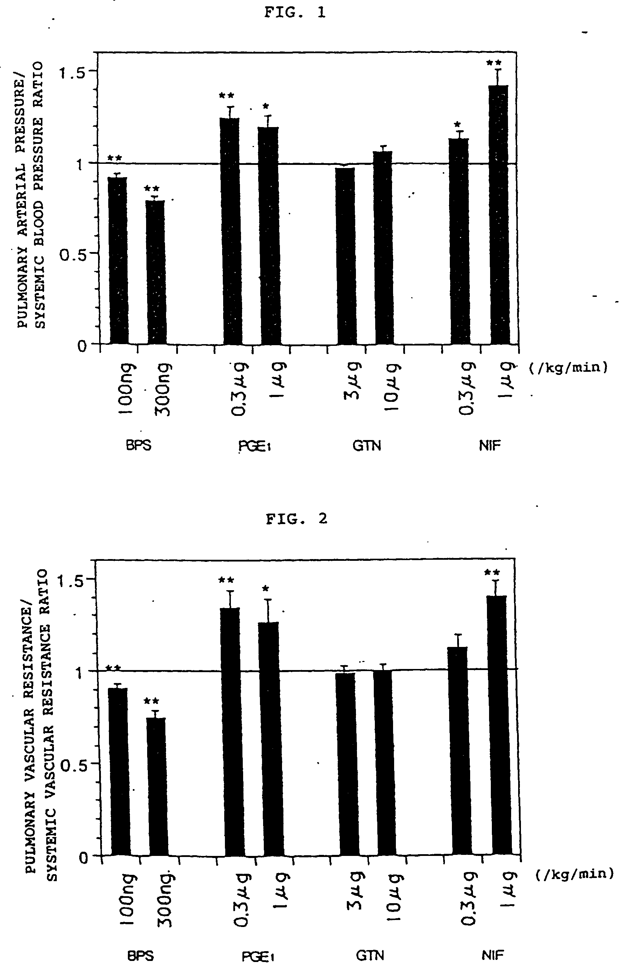

A thromboxane receptor agonist U-46619 was continuously injected into anesthetized dogs in a dose of 0.3 µg/kg/min to increase the pulmonary arterial pressure. Beraprost sodium (100, 300 ng/kg/min) and prostaglandin E₁ (0.3, 1 µg/kg/min), nitroglycerin (3, 10 µg/kg/min) and nifedipine (0.3, 1 µg/kg/min) were continuously intravenously administered in a dose causing the same degree of decrease in blood pressure as beraprost sodium to examine decreases in the pulmonary arterial pressure and the systemic blood pressure and decreases in the pulmonary vascular resistance and the systemic vascular resistance. Fig. 1 shows changes in the pulmonary arterial pressure/systemic blood pressure ratio, and Fig. 2 shows changes in the pulmonary vascular resistance/systemic vascular resistance ratio. In Figs. 1 and 2, BPS represents beraprost sodium, PGE₁ represents prostaglandin E₁, GTN represents nitroglycerin, and NIF represents nifedipine. In each of the figures, the ratio on the ordinate are based on a value of 1 before administration of medicines. Marks "*" and "**" represent comparisons with values before administration of medicines with p < 0.05 and 0.01, respectively (paired t-test).

Both figures indicate that beraprost sodium significantly decreases the pulmonary arterial pressure/systemic blood pressure ratio and the pulmonary vascular resistance/systemic vascular resistance ratio, and selectively dilates the pulmonary blood vessels. On the other hand, such an action was not observed in prostaglandin E₁, nitroglycerin and nifedipine.

### Industrial Applicability

The pulmonary circulation improving agent of the present invention has the dilating action selective to the pulmonary blood vessels, and the present invention provides a pulmonary circulation improving agent having excellent effectiveness and practicability.

## Claims

1. A pulmonary circulation improving agent containing a prostaglandin I derivative as an active component.

2. The pulmonary circulation improving agent according to Claim 1, wherein the prostaglandin I derivative is a prostaglandin I₂ derivative.

3. The pulmonary circulation improving agent according to Claim 1, wherein the prostaglandin I derivative is a 4,8-inter-m-phenylene prostaglandin I₂ derivative or a pharmacologically acceptable salt thereof represented by the following formula (I): 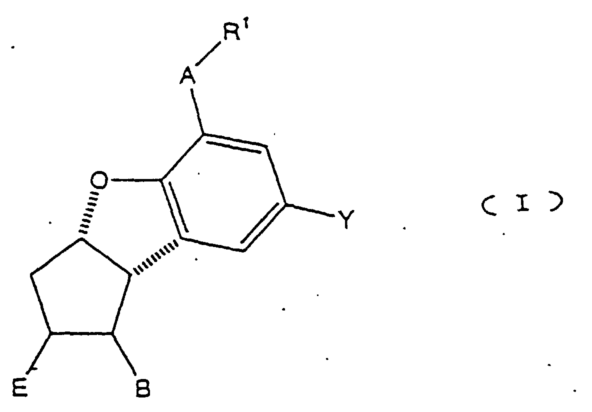 [wherein R¹ is the following:
(A) COOR² wherein R² is:
1) hydrogen or pharmacologically acceptable cation;
2) straight chain alkyl having 1 to 12 carbon atoms or branched alkyl having 3 to 14 carbon atoms;
3) -Z-R³
wherein Z is a valence bond or straight chain or branched alkylene represented by CₜH₂ₜ wherein t represents an integer of 1 to 6, and R³ is cycloalkyl having 3 to 12 carbon atoms or substituted cycloalkyl having 3 to 12 carbon atoms and substituted by 1 to 3 R⁴ groups which are each hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein the substituent is at least one chlorine, bromine, fluorine, iodine, trifluoromethyl, alkyl having 1 to 4 carbon atoms, nitro, cyano, methoxy, phenyl, phenoxy, p-acetoamidobenzamido, -CH=N-NH-C(=O)-NH₂, -NH-C(=O)-Ph, -NH-C(=O)-CH₃ or -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9) -CₚH₂ₚ-W-R⁷
wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the same as the above in (A) 5)), or aralkyl having 7 to 12 carbon atoms, and two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
wherein m represents an integer of 1 to 3;
Y is hydrogen, alkyl having 1 to 4 carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-; or
3) -C≡C-; and
R¹² is the following:
1) straight chain alkyl having 1 to 12 carbon atoms, or branched alkyl having 3 to 14 carbon atoms;
2) -Z-Ar²
wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents straight chain or branched alkylene, and R¹⁷ represents straight chain alkyl having 1 to 6 carbon atoms;
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form].

4. The pulmonary circulation improving agent according to Claim 1, wherein the prostaglandin I derivative is beraprost or a salt thereof.

5. The pulmonary circulation improving agent according to Claim 1, wherein the prostaglandin I derivative is ataprost, iloprost, clinprost, ciprostene, naxaprostene, taprostene, cicaprost, pimilprost, CH-169, or CS570.

6. A pulmonary circulation improving method comprising administering a patient suffering from an increase in the pulmonary vascular resistance with an agent containing a prostaglandin I derivative as an active component.

7. The pulmonary circulation improving method according to Claim 6, comprising administering the prostaglandin I derivative 1 to 3 times a day in a dose of 0.01 to 100 mg/adult.

8. The pulmonary circulation improving method according to Claim 6, wherein the prostaglandin I derivative is a prostaglandin I₂ derivative.

9. The pulmonary circulation improving method according to Claim 6, wherein the prostaglandin I derivative is a 4,8-inter-m-phenylene prostaglandin I₂ derivative or a pharmacologically acceptable salt thereof represented by the following formula (I): 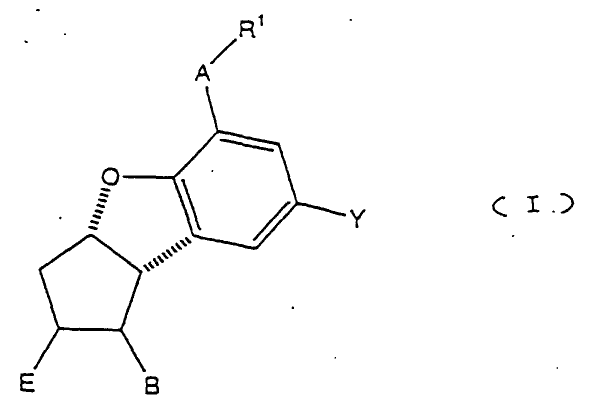 [wherein R¹ is the following:
(A) COOR² wherein R² is:
1) hydrogen or pharmacologically acceptable cation;
2) straight chain alkyl having a carbon number of 1 to 12 or branched alkyl having 3 to 14 carbon atoms;
3) -Z-R³
wherein Z is a valence bond or straight chain or branched alkylene represented by CₜH₂ₜ wherein t represents an integer of 1 to 6, and R³ is cycloalkyl having 3 to 12 carbon atoms or substituted cycloalkyl having 3 to 12 carbon atoms and substituted by 1 to 3 R⁴ groups which are each hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein the substituent is at least one chlorine, bromine, fluorine, iodine, trifluoromethyl, alkyl having 1 to 4 carbon atoms, nitro, cyano, methoxy, phenyl, phenoxy, p-acetoamidobenzamido, -CH=N-NH-C(=O)-NH₂, -NH-C(=O)-Ph, -NH-C(=O)-CH₃ or -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=O)-R⁶
wherein R⁵ is hydrogen or benzoyl, and R⁶ is phenyl, p-bromophenyl, p-chlorophenyl, p-biphenyl, p-nitrophenyl, p-benzamidopnenyl, or 2-naphthyl;
9) -CₚH₂ₚ-W-R⁷
wherein W is -CH=CH-, -CH=CR⁷ or -C≡C-, and R⁷ is hydrogen or straight chain or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) -CH₂OH;
(C) -C(=O)N(R⁹)₂
wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substitutent is defined as the same as the above in (A) 5)), or aralkyl having 7 to 12 carbon atoms, and two R⁹ groups may be the same or different, and when one of the R⁹ groups is -SO₂R¹⁰, the other R⁹ is not -SO₂R¹⁰; or
(D) -CH₂OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C≡C-;
wherein m represents an integer of 1 to 3;
Y is hydrogen, alkyl having 1 to 4 carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxyethyl, or t-butyl; X is the following:
1) -CH₂-CH₂-;
2) -CH=CH-;
3) -C≡C-; and
R¹² is the following:
1) straight chain alkyl having 1 to 12 carbon atoms, or branched alkyl having 3 to 14 carbon atoms;
2) -Z-Ar²
wherein Z is the defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, methoxy, phenyl or phenoxy, cyclopentyl, cyclohexyl, or cyclopentyl or cyclohexyl substituted by 1 to 4 straight chain alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C≡C-R¹⁷
wherein u is an integer of 1 to 7, CᵤH₂ᵤ represents straight chain or branched alkylene, and R¹⁷ represents straight chain alkyl having 1 to 6 carbon atoms;
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents the d, l or dl form].

10. The pulmonary circulation improving method according to Claim 6, wherein the prostaglandin I derivative is beraprost or a salt thereof.

11. The pulmonary circulation improving method according to Claim 6, wherein the prostaglandin I derivative is ataprost, iloprost, clinprost, ciprostene, naxaprostene, taprostene, cicaprost, pimilprost, CH-169, or CS570.
